# FASCICULE DE BREVET EUROPEEN

(11) **EP 3 169 655 B1**
(45) Date de publication et mention de la délivrance du brevet: **06.03.2019**
(21) Numéro de dépôt: 15728509.9
(22) Date de dépôt: 10.06.2015
(51) Int. Cl.: C07C 5/27, C07C 7/13, C07C 15/08

(54) **PROCÉDÉ DE PRODUCTION DE PARAXYLÈNE À HAUTE PURETÉ À PARTIR D'UNE COUPE XYLÈNE, PROCÉDÉ UTILISANT UNE UNITÉ DE SÉPARATION EN LIT MOBILE SIMULÉ ET DEUX UNITÉS D'ISOMÉRISATION, L'UNE EN PHASE GAZ L'AUTRE EN PHASE LIQUIDE**
VERFAHREN ZUR HERSTELLUNG VON HOCHREINEM PARAXYLOL AUS EINER XYLOLFRAKTION, VERFAHREN MIT EINER SIMULIERTEN WANDERBETTTRENNEINHEIT UND ZWEI ISOMERISIERUNGSEINHEITEN, EINE DAVON IN DER GASPHASE UND DIE ANDERE IN DER FLÜSSIGPHASE
METHOD FOR THE PRODUCTION OF HIGH-PURITY PARAXYLENE FROM A XYLENE FRACTION, METHOD USING A SIMULATED MOVING BED SEPARATION UNIT AND TWO ISOMERISATION UNITS, ONE BEING IN THE GAS PHASE AND THE OTHER BEING IN THE LIQUID PHASE

(30) Priorité: 18.07.2014 FR 1456942
(43) Date de publication de la demande: 24.05.2017
(73) Titulaire: IFP Energies nouvelles, 92500 Rueil-Malmaison (FR)
(72) Inventeur: DREUX, Heloise, F-69007 Lyon (FR); LEFLAIVE, Philibert, F-69780 Mions (FR); LEINEKUGEL LE COCQ, Damien, F-69600 Oullins (FR)
(74) Mandataire: IFP Energies nouvelles
(86) Numéro de dépôt international: PCT/EP2015/062985
(87) Numéro de publication internationale: WO 2016/008654

(56) Documents cités:
- FR-A1- 2 862 638
- US-A1- 2014 155 667

## Description

### DOMAINE DE L'INVENTION

La production de paraxylène est en constante augmentation depuis trente ans. Les utilisations du paraxylène sont principalement les productions d'acide téréphtalique et de résines polyéthylène téréphtalate, pour fournir des textiles synthétiques, des bouteilles, et plus généralement des matières plastiques.

Pour satisfaire la demande toujours croissante en paraxylène, les pétrochimistes ont le choix entre pratiquer des augmentations de capacité sur des unités existantes ou construire des unités neuves.

La présente invention décrit un procédé de production de paraxylène à haute pureté qui peut aussi bien s'appliquer à des unités neuves qu'au dégoulottage d'unités existantes.

### EXAMEN DE L'ART ANTERIEUR

La production de paraxylène haute pureté par séparation par adsorption est bien connue de l'art antérieur. De manière industrielle, cette opération est réalisée au sein d'un enchainement de procédés dit « boucle C8-aromatique ». Cette « boucle C8-aromatique » inclut une étape d'élimination des composés lourds (i.e. C9+) dans une colonne de distillation appelée « colonne des xylènes ». Le flux de tête de cette colonne, qui contient les isomères en C8-aromatiques, est ensuite envoyé dans le procédé de séparation du paraxylène qui est très généralement un procédé de séparation par adsorption en lit mobile simulé.

L'extrait, qui contient le paraxylène est ensuite distillé (colonne d'extrait puis colonne toluène) pour obtenir du paraxylène de haute pureté. Le raffinat, riche en métaxylène, orthoxylène et éthylbenzène, après une étape d'élimination du solvant par distillation est traité dans une unité catalytique d'isomérisation qui redonne un mélange d'aromatiques en C8, dans lequel la proportion des xylènes (ortho-, méta-,para- xylènes) est pratiquement à l'équilibre thermodynamique et la quantité d'éthylbenzène amoindrie. Ce mélange est à nouveau envoyé dans la « colonne des xylènes » avec la charge fraiche.

Tous les procédés industriels d'isomérisation des C8-aromatiques permettent d'isomériser les xylènes. La transformation de l'éthylbenzène dépend, en revanche, du type de procédé et de catalyseur choisis. En effet, les complexes pétrochimiques utilisent une unité d'isomérisation dite « isomérisante » (i.e. isomérisant l'éthylbenzène en un mélange de C8 aromatiques) ou « désalkylante » (désalkylation de l'éthylbenzène en benzène), afin de privilégier la production respectivement soit de paraxylène seul, soit de benzène et paraxylène.

Le choix du catalyseur utilisé dépend de la transformation de l'éthylbenzène souhaitée. Lorsque la réaction cible est l'isomérisation de l'éthylbenzène, elle nécessite un catalyseur bifonctionnel présentant à la fois une fonction acide et une fonction hydrogénante.

Il a en effet été démontré que l'éthylbenzène est tout d'abord hydrogéné en éthylcyclohexène sur les sites métalliques, puis transformé en dimethylcyclohexène sur sites acides par contraction puis expansion de cycle, et enfin deshydrogéné en xylènes.

Lorsque la réaction cible est la désalkylation de l'éthylbenzène, elle se produit uniquement sur des sites acides. La présence d'une phase hydrogénante sur le catalyseur permet cependant d'hydrogéner immédiatement l'éthylène formé et d'obtenir une désalkylation totale, évitant ainsi toute réalkylation ultérieure. Dans les deux cas l'incorporation d'une phase métallique dans le catalyseur permet également d'assurer sa stabilité.

Les procédés industriels d'isomérisation utilisent donc des catalyseurs hétérogènes bifonctionnels (acides et métalliques) mis en oeuvre en lit fixe et opérant en phase vapeur sous pression d'hydrogène, dans des gammes de température comprises généralement entre 380-440°C et des pressions de 10 à 20 bar .

Le choix d'une isomérisation « isomérisante » permet, comme indiqué ci-dessus, de maximiser la production de paraxylène, qui est le composé ayant la plus forte valeur ajoutée en sortie du complexe aromatique. Cette solution présente cependant l'inconvénient de générer lors de l'étape d'isomérisation des pertes en cycles aromatiques par craquage plus importantes qu'avec une isomérisation désalkylante, le cycle étant transitoirement au moins partiellement hydrogéné.

Le choix du type d'isomérisation se présente donc comme un compromis entre d'une part la minimisation de la perte de cycles aromatiques associée à une coproduction de benzène, produit à plus faible valeur ajoutée que le paraxylène (isomérisation désalkylante), et d'autre part une maximisation de la production de paraxylène qui présente l'inconvénient de générer des pertes en cycles aromatiques plus importante (isomérisation « isomérisante »).

Il existe donc un besoin pour un schéma de procédé permettant à la fois une maximisation de la quantité de paraxylène produite avec une perte en cycles aromatiques réduite.

Plusieurs solutions sont proposées dans l'art antérieur afin d'atteindre cette objectif, celles-ci mettant généralement en oeuvre une isomérisation (de préférence désalkylante) associée à des étapes de conversion du benzène par transalkylation et/ou de méthylation du toluène ou du benzène tels que par exemple US2013/0267746, .

Il a été découvert de manière surprenante, que l'association au sein d'un complexe aromatique d'une isomérisation « isomérisante » et d'une isomérisation en phase liquide telle que décrite par exemple dans les brevets US2011/263918, US7371913, US4962258 et US6180550 permettait de maximiser la quantité de paraxylène produite tout en ayant une perte en cycles aromatiques réduite par rapport à un complexe aromatique selon l'art antérieur.

Le document US 2014/0155667 décrit un procédé de production de paraxylène comportant une unité de séparation des xylènes et deux unités d'isomérisation combinées de manière à réduire le recyclage des xylènes.

Aucune condition opératoire n'est fournie pour les unités d'isomérisation. Le document FR 2 862 638 décrit un procédé de production de paraxylène faisant également appel à une unité de séparation des xylènes et deux unités d'isomérisation, l'unité de séparation produisant deux raffinats. Dans ce document les conditions opératoires des deux unités d'isomérisation ne sont pas différenciées.

### DESCRIPTION SOMMAIRE DES FIGURES

La figure 1 représente un schéma du procédé selon la présente invention.
La figure 2 représente un schéma du procédé selon l'art antérieur.

Dans la suite du texte on désigne l'unité de séparation en lit mobile simulé par unité de séparation (SMB) et les deux unités d'isomérisation par (ISOM-1) et (ISOM-2). Les colonnes (S-1), (RAF) et (EXT) sont des colonnes de distillation.

Une unité de séparation en lit mobile simulé peut être constituée de plusieurs adsorbeurs.

### DESCRIPTION SOMMAIRE DE L'INVENTION

La présente invention peut se définir comme un procédé de production de paraxylène à haute pureté à partir d'une coupe xylènes contenant de l'éthylbenzène et des composés en C9+, procédé utilisant une unité de séparation en lit mobile simulé (SMB) et deux unités d'isomérisation, l'une (ISOM-1) travaillant en phase liquide, et l'autre (ISOM-2) travaillant en phase gaz.

Le procédé selon l'invention consiste en la suite d'étapes suivantes :
- on envoie la charge fraiche (1) en mélange avec l'isomérat (16) issu de l'unité d'isomérisation (ISOM-2) en phase gaz dans la colonne de distillation (S-1) de laquelle on sort en tête un flux (3) qui est mélangé avec le second isomérat (14) issu de l'unité d'isomérisation (ISOM-1) en phase liquide, et en fond un flux (4) constitué essentiellement de composés en C9 et C10 aromatiques et éventuellement d'orthoxylène,
- on effectue une séparation en lit mobile simulé du flux (5) résultant du mélange des flux (3) et (14) dans une unité de séparation (SMB) comportant au moins un adsorbeur contenant une pluralité de lits interconnectés et travaillant en boucle fermée, ladite unité de séparation comprenant au moins quatre zones définies de la façon suivante :
   - la zone 1 comprise entre l'injection du désorbant (11) et le soutirage de l'extrait (6),
   - la zone 2 comprise entre le soutirage de l'extrait (6) et l'injection de la charge (5),
   - la zone 3 comprise entre l'injection de la charge (5) et le soutirage du raffinat (9),
   - la zone 4 comprise entre le soutirage du raffinat (9) et l'injection du désorbant (11).
- on envoie l'extrait (6) dans une colonne à distiller (EXT) de laquelle on soutire en tête un mélange de paraxylène et de toluène par la ligne (7), et en fond le désorbant (8) qui est renvoyé dans l'unité de séparation (SMB) par la ligne (11),
- on envoie le raffinat (9) dans une colonne à distiller (RAF) de laquelle on soutire en fond le désorbant (10) qui est renvoyé dans l'unité de séparation (SMB) par la ligne (11), et en tête un mélange de métaxylène, d'orthoxylène et d'éthylbenzène qui est envoyé par une ligne (12), vers les unités d'isomérisation (ISOM-1 et ISOM-2),
- on envoie dans l'unité d'isomérisation en phase liquide (ISOM-1) une première partie du flux (12), notée flux (13), pour obtenir un premier isomérat (14) alimentant en partie l'unité de séparation en lit mobile simulé (SMB),
- on envoie dans l'unité d'isomérisation en phase gaz (ISOM-2) une seconde partie du flux (12), notée flux (15), pour obtenir un isomérat (16) qui est envoyé en mélange avec la charge fraiche (1) dans la colonne à distiller (S-1).

L'unité d'isomérisation en phase gaz (ISOM-2) fonctionne aux conditions suivantes :
- température supérieure à 300°C, de préférence de 350°C à 480°C,
- pression inférieure à 4,0 MPa, et de préférence de 0,5 à 2,0 MPa,
- vitesse spatiale inférieure à 10 h⁻¹, de préférence comprise entre 0,5 h⁻¹ et 6 h⁻¹,
- rapport molaire hydrogène sur hydrocarbure inférieur à 10, et de préférence compris entre 3 et 6,
et le catalyseur utilisé dans ladite unité d'isomérisation ISOM-2 comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids, bornes incluses.
l'unité d'isomérisation (ISOM-1) fonctionne en phase liquide aux conditions suivantes :
- Température inférieure à 300°C, de préférence 200°C à 260°C
- Pression inférieure à 4 MPa, de préférence 2 à 3 MPa
- Vitesse spatiale horaire (VVH) inférieure à 10h⁻¹ (10 litres par litre et par heure), de préférence comprise entre 2 et 4 h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée un catalyseur comportant une zéolithe de type ZSM-5.

Selon une autre variante préférée du procédé de production de paraxylène à haute pureté selon l'invention, le catalyseur utilisé dans l'unité d'isomérisation (ISOM-2) renferme de 1% à 70% poids d'une zéolithe de type structural EUO (EU-1 par exemple) comprenant du silicium et au moins un élément T choisi de préférence parmi l'aluminium et le bore dont le rapport Si/T est compris entre 5 et 100.

Selon une autre variante préférée du procédé de production de paraxylène à haute pureté selon l'invention, la zéolithe faisant partie du catalyseur de l'unité d'isomérisation (ISOM-2) est sous forme hydrogène au moins en partie, et la teneur en sodium est telle que le ratio atomique Na/T est inférieur à 0,1.

Selon une autre variante préférée du procédé de production de paraxylène à haute pureté selon l'invention, le catalyseur de l'unité d'isomérisation (ISOM-2) peut contenir entre 0,01 et 2% poids d'étain ou d'indium, et du soufre à raison de 0,5 à 2 atomes par atome de métal du groupe VIII.

Selon une autre variante préférée du procédé de production de paraxylène à haute pureté selon l'invention, le nombre total de lits de l'unité de séparation (SMB) est compris entre 6 et 24 lits, et de manière préférée, entre 8 et 15 lits répartis sur un ou plusieurs adsorbeurs, le nombre de lits étant ajusté de manière à ce que chaque lit ait de une hauteur comprise entre 0,70 m et 1,40 m.

Selon une autre variante préférée du procédé de production de paraxylène à haute pureté selon l'invention, la répartition de la quantité de solide adsorbant dans chaque zone de l'unité de séparation (SMB) est la suivante :
- la quantité de solide adsorbant en zone 1 est de 17%±5%,
- la quantité de solide adsorbant en zone 2 est de 42%±5%,
- la quantité de solide adsorbant en zone 3 est de 25%±5%,
- la quantité de solide adsorbant en zone 4 est de 17%±5%,

Selon une autre variante préférée du procédé de production de paraxylène à haute pureté selon l'invention, on injecte le désorbant et la charge dans l'unité de séparation (SMB) dans un rapport volumétrique désorbant sur charge d'au plus 1,7/1, et de manière préférée compris entre 1,5/1 et 0,4/1, bornes incluses.

Selon une autre variante préférée du procédé de production de paraxylène à haute pureté selon l'invention, on extrait de l'unité de séparation (SMB) non plus un seul raffinat (9) mais deux raffinats distincts (R1) et (R2), c'est-à-dire prélevés en deux points différents de l'unité SMB, (R1) étant envoyé à l'unité d'isomérisation (ISOM-1) et (R2) étant envoyé dans l'unité d'isomérisation (ISOM-2).

### DESCRIPTION DETAILLEE DE L'INVENTION

On mélange la charge (1) avec l'isomérat (16) pour former le flux (2). On envoie le flux (2) dans une colonne de distillation (S-1) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante d'orthoxylène.

On mélange le flux de tête (3) de la colonne à distiller (S-1) avec l'isomérat (14) pour former le flux (5).

On effectue une première séparation du mélange (5) dans une unité de séparation en lit mobile simulé (SMB) comprenant au moins un adsorbeur contenant une pluralité de lits interconnectés et travaillant en boucle fermée, ladite unité de séparation comprenant au moins quatre zones délimitées par les injections du flux (5) et du désorbant (11), et les soutirages d'un extrait (6) contenant du paraxylène et d'un raffinat (9) contenant de l'orthoxylène et du métaxylène.

On distille préférentiellement l'extrait (6) dans une colonne (EXT), pour récupérer une première fraction (7) enrichie en paraxylène.

On distille préférentiellement le raffinat (9) dans une colonne (RAF) pour éliminer sensiblement tout le désorbant et pour soutirer une fraction distillée (12).

Cette fraction distillée (12) est divisée en deux flux (13) et (15). Le flux (13) alimente une première unité d'isomérisation (ISOM-1) pour obtenir un premier isomérat (14) alimentant préférentiellement l'unité de séparation (SMB), mais pouvant être en partie recyclé en entrée de la colonne de distillation (S-1).

Le flux (15) alimente une deuxième unité d'isomérisation (ISOM-2) pour obtenir un deuxième isomérat (16) recyclé en entrée de la colonne de séparation (S-1).

Le désorbant utilisé dans l'unité de séparation (SMB) est généralement choisi parmi le paradiéthylbenzène, le toluène, le paradifluorobenzène ou des diéthylbenzènes en mélange. Le rapport volumique du désorbant sur la charge dans l'unité de séparation (SMB) est compris entre 0,5 et 2,5, et de préférence compris entre 0,8 et 2.

L'unité de séparation en lit mobile simulé (SMB) est opérée à une température comprise entre 20°C et 250°C, de préférence entre 90°C et 210 °C, et de manière encore préférée entre 140°C et 180°C, et sous une pression comprise entre la pression de bulle des xylènes à la température opératoire et 2 MPa.

La charge fraîche est introduite par la ligne (1) dans une colonne à distiller (S-1). Cette charge fraîche contient majoritairement des composés C8-aromatiques, xylènes et éthylbenzène, en proportion variable selon l'origine de la coupe. Elle peut contenir éventuellement des impuretés en quantité variable selon l'origine de la charge qui seront essentiellement des composés C9 et C10 aromatiques et des composés paraffiniques et naphténiques.

La teneur en impuretés naphténiques ou paraffiniques de la charge est avantageusement inférieure à 1% poids. De manière préférée, cette teneur est inférieure à 0,3% poids, et de manière encore préférée cette teneur est inférieure à 0,1 %poids.

La charge peut être issue, soit d'une unité de reformage, soit d'une unité de dismutation du toluène, soit d'une unité de transalkylation du toluène et des C9 aromatiques.

On ajoute à la charge fraîche un isomérat véhiculé par une ligne (16).

L'effluent de fond (4) de la colonne (S-1) est constitué essentiellement de composés en C9 et C10 aromatiques et éventuellement d'orthoxylène.

Optionnellement, le mélange (4) d'orthoxylène et d'hydrocarbures aromatiques en C9-C10 soutiré en fond de la colonne de distillation (S-1) peut être envoyé dans une autre colonne de distillation d'où l'on extrait en tête un flux d'orthoxylène de haute pureté (au moins 98,5 %), et en fond un flux contenant des hydrocarbures en C9-C10.

L'effluent de tête (3) de la colonne à distiller (S-1) est mélangé à l'isomérat (14) pour former le flux (5) qui constitue la charge d'une unité de séparation (SMB). L'unité de séparation (SMB) est alimentée d'une part par la charge véhiculée par la ligne (5), et d'autre part par du désorbant véhiculé par une ligne (11).

Les effluents de l'unité de séparation(SMB) sont un extrait (6) et un raffinat (9), ladite unité de séparation comprenant au moins quatre zones délimitées par les injections de charge et de désorbant, et les soutirages de raffinat et d'extrait,
- la zone 1 comprise entre l'injection du désorbant (11) et le soutirage de l'extrait (6),
- la zone 2 comprise entre le soutirage de l'extrait (6) et l'injection de la charge (5),
- la zone 3 comprise entre l'injection de la charge (5) et le soutirage du raffinat (9),
- la zone 4 comprise entre le soutirage du raffinat (9) et l'injection du désorbant (11).

Le nombre total de lits de l'unité de séparation (SMB) selon l'invention est de préférence compris entre 6 et 24 lits, et de manière encore plus préférée, entre 8 et 15 lits répartis sur un ou plusieurs adsorbeurs.

Le nombre de lits est ajusté de manière à ce que chaque lit ait de préférence une hauteur comprise entre 0,70 m et 1,40 m.

La répartition de la quantité de solide adsorbant dans chaque zone est la suivante :
- la quantité de solide adsorbant en zone 1 est de 17%±5%,
- la quantité de solide adsorbant en zone 2 est de 42%±5%,
- la quantité de solide adsorbant en zone 3 est de 25%±5%,
- la quantité de solide adsorbant en zone 4 est de 17%±5%,

Selon une caractéristique préférée de l'invention, on peut injecter le désorbant et la charge dans l'unité de séparation (SMB) dans un rapport volumétrique désorbant sur charge d'au plus 1,7/1, et de manière préférée compris entre 1,5/1 et 0,4/1, bornes incluses.

L'extrait (6) est constitué essentiellement de toluène, de paraxylène et de désorbant.

Le raffinat (9) est constitué essentiellement de toluène, de métaxylène, d'orthoxylène, d'éthylbenzène, de paraxylène pour la partie non récupérée dans l'extrait, et de désorbant. L'extrait (6) est envoyé dans une colonne à distiller (EXT).

En fond de la colonne à distiller (EXT) on soutire le désorbant (8) qui est renvoyé dans l'unité de séparation (SMB) par la ligne (11). En tête de la colonne à distiller (EXT), on soutire un mélange de paraxylène et de toluène par la ligne (7).

Le raffinat (9) est envoyé dans une colonne à distiller (RAF).

En fond de la colonne (RAF) on soutire du désorbant (10) qui est renvoyé dans l'unité de séparation (SMB) par la ligne (11). En tête de la colonne (RAF), on soutire un mélange de métaxylène, d'orthoxylène et d'éthylbenzène par une ligne (12), que l'on envoie vers les unités d'isomérisation (ISOM-1) et (ISOM-2).

Le flux 12 est divisé en deux flux (13) et (15), respectivement dans les proportions variant entre 10-90 et 90-10, préférentiellement entre 25-75 et 75-25, ces proportions étant des pourcentages massiques.

La première zone d'isomérisation (ISOM-1) travaille de en phase liquide et est opérée dans les conditions suivantes :
- Température inférieure à 300°C, de préférence 200°C à 260°C
- Pression inférieure à 4 MPa, de préférence 2 à 3 MPa
- Vitesse spatiale horaire (VVH) inférieure à 10h⁻¹ (10 litres par litre et par heure), de préférence comprise entre 2 et 4 h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée un catalyseur comportant une zéolithe de type ZSM-5.

L'effluent de l'unité d'isomérisation (ISOM-1) est renvoyé par la ligne (14) soit vers la colonne à distiller (S-1), soit directement à l'entrée de l'unité de séparation (SMB) dans le cas où la teneur en composés autres que les C8-aromatiques est très faible, typiquement de l'ordre de 1% poids. La teneur en C9 est typiquement inférieure à 1000 ppm pds.

La deuxième unité d'isomérisation (ISOM-2) travaille en phase gazeuse et est généralement opérée dans les conditions suivantes :
- Température supérieure à 300°C, de préférence 350°C à 480°C
- Pression inférieure à 4 MPa, de préférence 0,5 à 2 MPa
- Vitesse spatiale horaire (VVH) inférieure à 10h⁻¹ (10 litres par litre et par heure), de préférence comprise entre 0,5 et 6 h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant une zéolithe de type structural EUO ou MOR, et au moins un métal du groupe VIII
- Rapport molaire H₂/hydrocarbures inférieur à 10, de préférence compris entre 3 et 6.

Tous les catalyseurs susceptibles d'isomériser les hydrocarbures à 8 atomes de carbone, zéolithiques ou non, conviennent pour l'unité d'isomérisation (ISOM-2) de la présente invention. De préférence, on utilise un catalyseur contenant une zéolithe acide, par exemple de type structural MFI, MOR, MAZ, FAU et/ou EUO. De manière encore plus préférée, on utilise un catalyseur contenant une zéolithe de type structural EUO et au moins un métal du groupe VIII de la classification périodique des éléments.

De manière préférée, le catalyseur de l'unité d'isomérisation (ISOM-2) renferme de 1% à 70% poids d'une zéolithe de type structural EUO (EU-1 par exemple) comprenant du silicium et au moins un élément T choisi de préférence parmi l'aluminium et le bore dont le rapport Si/T est compris entre 5 et 100. La dite zéolithe est sous forme hydrogène au moins en partie, et la teneur en sodium est telle que le ratio atomique Na/T est inférieur à 0,1. Eventuellement le catalyseur de l'unité d'isomérisation peut contenir entre 0,01 et 2% poids d'étain ou d'indium, et du soufre à raison de 0,5 à 2 atomes par atome de métal du groupe VIII.

L'effluent de l'unité d'isomérisation (ISOM-2) est envoyé dans un train de séparations qui permet de récupérer une partie de l'hydrogène que l'on recycle à l'unité d'isomérisation (ISOM-2). La partie d'hydrogène non recyclé est compensée par un appoint d'hydrogène frais. On récupère à l'issue du train de séparation un isomérat constitué des fractions les plus lourdes qui est renvoyé vers la colonne à distiller (S-1) par la ligne (16).

### EXEMPLES SELON L'ART ANTERIEUR ET SELON L'INVENTION

### Exemple 1 (selon l'art antérieur):

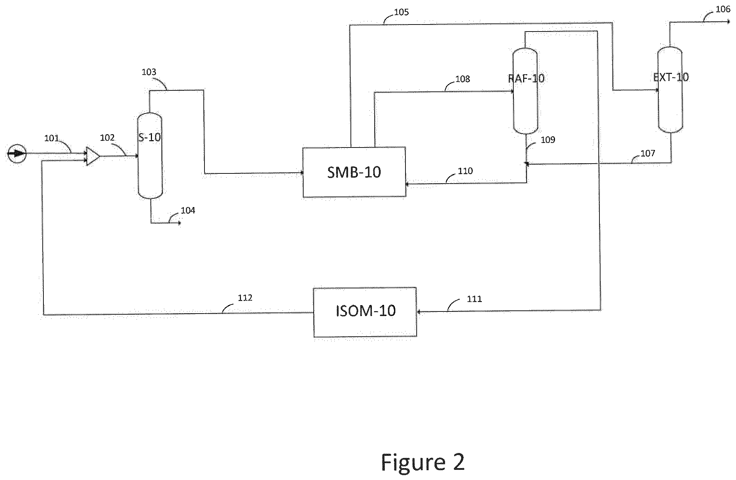

Cet exemple illustre l'art antérieur et décrit un complexe aromatique tel que schématisé sur la figure 2 et comportant :
- une colonne des xylènes (S-10) permettant d'extraire les aromatiques en C9 et C10 (flux 104) et d'envoyer à l'unité de séparation (SMB-10) un flux (103) constitué essentiellement d'aromatiques en C8,
- une unité de séparation en lit mobile simulé (SMB-10) à 4 zones de laquelle on soutire un extrait (105) et un unique raffinat (108),
- une unité d'isomérisation (ISOM-10) alimentée par une partie (111) du raffinat (108) après élimination du désorbant (109) au moyen de la colonne à distiller (RAF-10).
- une colonne d'extrait du paraxylène (EXT-10) dont on soutire en fond le désorbant qu'on recycle à l'adsorption (SMB-10) via le flux (110), et en tête une coupe riche en paraxylène (106).

Le bilan matière du procédé est décrit dans le tableau 1 ci-dessous. Seuls les composés C8-aromatiques et C9+ sont décrits. On néglige les autres composés et la formation de C9+ dans les unités d'isomérisation. On utilise comme unité de débit le millier de tonnes par an (kt/an).

**TABLEAU 1**

| | | PX | EB | MOX | C9+ | Total |
|---|---|---|---|---|---|---|
| Charge fraîche | 101 | 23,6 | 15,6 | 67,7 | 13,8 | 120,6 |
| Charge S-10 | 102 | 100 | 45,9 | 297 | 13,8 | 456,6 |
| Charge SMB-10 | 103 | 100 | 45,9 | 297 | 0 | 442,9 |
| Fond S-10 | 104 | 0 | 0 | 0 | 13,8 | 13,8 |
| Tête EXT-10 | 106 | 100 | 0 | 0 | 0 | 100 |
| Charge ISOM-10 | 11 | 0 | 45,9 | 297 | 0 | 342,9 |
| Sortie ISOM-10 | 112 | 76,4 | 30,2 | 229,3 | 0 | 336,0 |

La charge (101) alimente la boucle aromatique (mélange du réformat lourd et du fond de la colonne de toluène) présente avec un débit de 120,4 kt/an. On adjoint à la charge (101) 336 kt/an d'isomérat (112) recyclés de l'unité d'isomérisation (ISOM-10) isomérisant l'éthylbenzène. Le flux résultant (102) est distillé dans la colonne de xylènes (S-10).

On soutire en fond de la colonne (S-10) 13,8 kt/an d'un mélange de C9 et C10 aromatiques (104) et en tête 442,9 kt/an de coupe C8 aromatiques (103) dont la teneur en paraxylène est de 22,6%, la teneur en éthylbenzène de 10,4%, la teneur en orthoxylène et en métaxylène de 67%.

Cette coupe est envoyée dans une unité de séparation en lit mobile simulé à quatre zones (SMB-10) et quatre flux principaux : la charge (103), le désorbant (110), l'extrait (105) et le raffinat (108). Cette unité de séparation est composée de 12 lits contenant une zéolithe X échangée au baryum. La température est de 175°C.

La configuration est :
- 2 lits en zone 1,
- 5 lits en zone 2,
- 3 lits en zone 3,
- 2 lits en zone 4.

Le solvant utilisé est le paradiéthylbenzène.

L'extrait (105) en sortie de l'unité d'adsorption (SMB-10) est envoyé dans une colonne à distiller (EXT-10) de laquelle on soutire en fond le désorbant recyclé vers l'unité de séparation (SMB-10), et en tête 100 kt/an d'un mélange (106) essentiellement constitué de toluène et de paraxylène.

Le raffinat est envoyé dans une colonne à distiller (RAF-10) de laquelle on soutire en fond le désorbant recyclé vers l'unité d'adsorption (SMB-10), et en tête 342,9 kt/an d'un mélange (111).

Ce flux est envoyé dans une unité d'isomérisation (ISOM-10).

L'unité d'isomérisation (ISOM-10) travaille en phase gazeuse aux conditions suivantes :
Température : 385°C
Catalyseur : contient du platine et de la zéolithe EU-1
Vitesse spatiale : 3,5 h⁻¹
Rapport H2/hydrocarbures : 4,4:1
Pression : 0,9 MPa

La teneur en éthylbenzène du mélange introduit dans l'unité d'isomérisation (ISOM-10) est de 13,4%.

On observe 2% de perte par craquage dans cette isomérisation soit un débit de 6,9 kt/an. L'éthylbenzène est isomérisé en partie, il en reste 9% dans le flux de sortie (112).

L'isomérat (112) présente un débit de 196 kt/an. Il est recyclé en entrée de la colonne (S-10) où il est mélangé avec la charge fraîche (101) qui présente un débit de 120,9 kt/an.

### Exemple 2 (selon l'invention) :

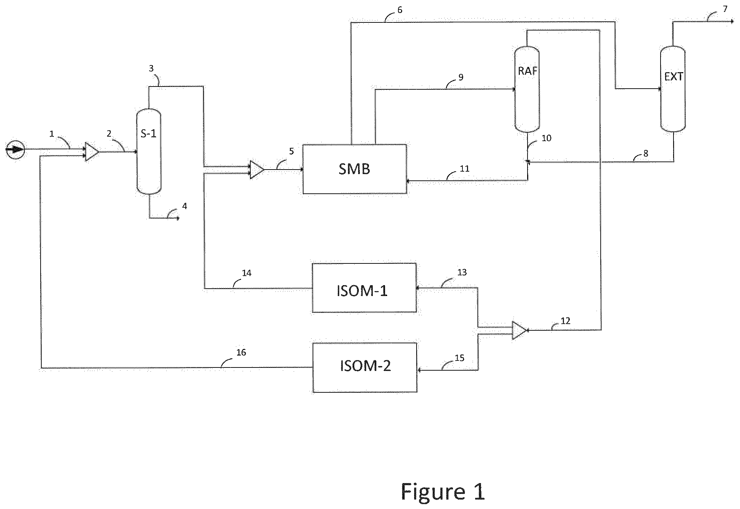

Cet exemple illustre l'invention et décrit une boucle aromatique schématisée sur la figure 1 et comportant :
- une colonne des xylènes (S-1) permettant d'extraire les aromatiques en C9 et C10 (4) et de récupérer en tête un flux (3) constitué essentiellement d'aromatiques en C8.
- une première unité d'adsorption en lit mobile simulé (SMB) à 4 zones de laquelle on soutire un extrait (6) et un raffinat (9).
- une première colonne d'extrait du paraxylène (EXT) dont on soutire en fond le désorbant (8) qu'on recycle à l'unité d'adsorption (SMB) via le flux (11), et en tête une coupe riche en paraxylène (7).
- une première unité d'isomérisation (ISOM-1) alimentée par une première partie du raffinat (9) après élimination du désorbant (10) au moyen de la colonne à distiller (RAF).
- une deuxième unité d'isomérisation (ISOM-2) alimentée par une deuxième partie du raffinat (9) après élimination du désorbant (10) au moyen de la colonne à distiller (RAF).

Le bilan matière du procédé est décrit dans le tableau 2 ci-dessous. Seuls les composés C8-aromatiques et C9+ sont décrits. On néglige les autres composés et la formation de C9+ dans les unités d'isomérisation. On utilise comme unité de débit le millier de tonnes par an (kt/an).

**TABLEAU 2**

| | | PX | EB | MOX | C9+ | Total |
|---|---|---|---|---|---|---|
| Charge fraîche | 1 | 22,8 | 15,3 | 65,5 | 13,4 | 117 |
| Charge S-1 | 2 | 59,9 | 46,5 | 176,8 | 13,4 | 296,6 |
| Tête S-1 | 3 | 59,9 | 46,5 | 176,8 | 0 | 283,3 |
| Fond S-1 | 4 | 0 | 0 | 0 | 13,4 | 134 |
| Charge SMB | 5 | 100 | 62,3 | 297 | 0 | 459,3 |
| Tête EXT | 7 | 100 | 0 | 0 | 0 | 100 |
| Tête RAF | 12 | 0 | 62,3 | 297 | 0 | 359,3 |
| Charge ISOM-1 | 13 | 0 | 31,2 | 148,5 | 0 | 179,7 |
| Sortie ISOM-1 | 14 | 37,1 | 31,2 | 111,4 | 0 | 179,7 |
| Charge ISOM-2 | 15 | 0 | 31,2 | 148,5 | 0 | 179,7 |
| Sortie ISOM-2 | 16 | 40,1 | 15,8 | 120,2 | 0 | 176,1 |

La charge fraîche (1) qui alimente la boucle aromatique présente un débit de 117 kt/an.

On adjoint à cette charge 176,1 kt/an d'isomérat (16) recyclés de l'unité d'isomérisation (ISOM-2) isomérisant l'éthylbenzène. Le flux résultant (2) est distillé dans la colonne de xylènes (S-1).

On soutire en fond de la colonne (S-1) 13,4 kt/an d'un mélange de C9 et C10 aromatiques (4) et en tête 283,3 kt/an de coupe C8 aromatiques (3).

On adjoint à cette coupe C8 aromatiques (3) 179,7 kt/an d'isomérat (14) recyclés de l'unité d'isomérisation (ISOM-1).

On obtient un mélange (5) dont la teneur en paraxylène est de 21,8%, la teneur en éthylbenzène de 13,6%, la teneur en orthoxylène et en métaxylène de 64,6%.

Cette coupe est envoyée dans une unité d'adsorption en lit mobile simulé à quatre zones (SMB) et quatre flux principaux : la charge (5), le désorbant (11), l'extrait (6) et le raffinat (9). Cette unité est composée de 12 lits contenant une zéolithe X échangée au baryum.

La température est de 175°C. La configuration est : 2 lits en zone 1, 5 lits en zone 2, 3 lits en zone 3 et 2 lits en zone 4. Le solvant utilisé est le paradiéthylbenzène.

L'extrait (6) en sortie de l'unité d'adsorption (SMB) est envoyé dans une colonne à distiller (EXT) de laquelle on tire en fond le désorbant (8) recyclé vers l'unité d'adsorption (SMB), et en tête 100 kt/an d'un mélange (7) essentiellement constitué de toluène et de paraxylène.

Le raffinat (9) est envoyé dans une colonne à distiller (RAF) de laquelle on tire en fond le désorbant (10) recyclé vers l'unité d'adsorption (SMB), et en tête 359,3 kt/an d'un mélange (12).

Ce flux est divisé en deux flux égaux (13) et (15) de 179,7 kt/an chacun.

Le flux (13) est envoyé dans une unité d'isomérisation (ISOM-1).

L'unité d'isomérisation (ISOM-1) travaille en phase liquide aux conditions suivantes:
Température : 240°C
Catalyseur : contient de la zéolithe ZSM-5
Vitesse spatiale : 3 h⁻¹
Pression : 1,9 MPa

La teneur en éthylbenzène du mélange introduit dans l'unité d'isomérisation (ISOM-1) est de 17,3%. L'éthylbenzène n'est pas converti, sa quantité est donc la même dans le flux de sortie (14). Cet isomérat (14) présente un débit de 179,7 kt/an. Il est recyclé en entrée de l'unité d'adsorption (SMB) sans passer par la colonne (S-1).

Le flux (15) est envoyé dans une unité d'isomérisation (ISOM-2).

L'unité d'isomérisation (ISOM-2) travaille en phase gaz aux conditions suivantes :
Température : 385°C
Catalyseur : contient du platine et de la zéolithe EU-1
Vitesse spatiale : 3,5 h⁻¹
Pression : 0,9 MPa

La teneur en éthylbenzène du mélange introduit dans l'unité d'isomérisation (ISOM-2) est de 17,3%. On observe 2% de perte par craquage dans cette isomérisation, soit un débit de 3,6 kt/an.

L'éthylbenzène est isomérisé en partie. Il en reste 9% dans le flux de sortie (16).

Cet isomérat (16) présente un débit de 176,1 kt/an. Il est recyclé en entrée de la colonne (S-1 où il est mélangé avec la charge fraîche (1) qui présente un débit de 117 kt/an.

L'invention présente plusieurs avantages par rapport à l'art antérieur :
Tout d'abord l'isomérisation en phase liquide est moins énergivore que l'isomérisation en phase gaz. En effet, elle travaille à température plus faible. Elle travaille également sans recycle d'hydrogène donc sans compresseur de recycle. Enfin, elle produit beaucoup moins de sous-produits, notamment des aromatiques en C9, ce qui permet de by passer la colonne d'élimination des aromatiques en C9 (S-1), induisant une très forte baisse de l'énergie nécessaire à cette séparation. Le fait de coupler une isomérisation en phase liquide à une isomérisation en phase gaz permet de diminuer les pertes par craquage au sein de l'isomérisation en phase gaz. En effet pour sortir 100 kt/an de paraxylène il faut introduire 117 kt/an de charge fraîche dans l'invention contre 120,6 kt/an dans l'art antérieur.

## Revendications

1. Procédé de production de paraxylène à haute pureté à partir d'une coupe xylènes contenant de l'éthylbenzène et des composés en C9+, procédé utilisant une unité de séparation en lit mobile simulé (SMB) et deux unités d'isomérisation, l'une (ISOM-1) travaillant en phase liquide, et l'autre (ISOM-2) travaillant en phase gaz, le procédé consistant en la suite d'étapes suivantes :
- on envoie la charge fraiche (1) en mélange avec l'isomérat (16) issu de l'unité d'isomérisation (ISOM-2) en phase gaz dans la colonne de distillation (S-1) de laquelle on sort en tête un flux (3) qui est mélangé avec le second isomérat (14) issu de l'unité d'isomérisation (ISOM-1) en phase liquide, et en fond un flux (4) constitué essentiellement de composés en C9 et C10 aromatiques et éventuellement d'orthoxylène,
- on effectue une séparation en lit mobile simulé du flux (5) résultant du mélange des flux (3) et (14) dans une unité de séparation (SMB) comportant au moins un adsorbeur contenant une pluralité de lits interconnectés et travaillant en boucle fermée, ladite unité de séparation comprenant au moins quatre zones définies de la façon suivante :
- la zone 1 comprise entre l'injection du désorbant (11) et le soutirage de l'extrait (6),
- la zone 2 comprise entre le soutirage de l'extrait (6) et l'injection de la charge (5),
- la zone 3 comprise entre l'injection de la charge (5) et le soutirage du raffinat (9),
- la zone 4 comprise entre le soutirage du raffinat (9) et l'injection du désorbant (11).
- on envoie l'extrait (6) dans une colonne à distiller (EXT) de laquelle on soutire en tête un mélange de paraxylène et de toluène par la ligne (7), et en fond le désorbant (8) qui est renvoyé dans l'unité de séparation (SMB) par la ligne (11),
- on envoie le raffinat (9) dans une colonne à distiller (RAF) de laquelle on soutire en fond le désorbant (10) qui est renvoyé dans l'unité de séparation (SMB) par la ligne (11), et en tête un mélange de métaxylène, d'orthoxylène et d'éthylbenzène qui est envoyé par une ligne (12), vers les unités d'isomérisation (ISOM-1 et ISOM-2),
- on envoie dans l'unité d'isomérisation en phase liquide (ISOM-1) une première partie du flux (12), notée flux (13), pour obtenir un premier isomérat (14) alimentant en partie l'unité de séparation en lit mobile simulé (SMB),
ladite unité d'isomérisation (ISOM-1) fonctionnant en phase liquide aux conditions suivantes :
- Température inférieure à 300°C, de préférence 200°C à 260°C
- Pression inférieure à 4 MPa, de préférence 2 à 3 MPa
- Vitesse spatiale horaire (VVH) inférieure à 10h⁻¹ (10 litres par litre et par heure), de préférence comprise entre 2 et 4 h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée un catalyseur comportant une zéolithe de type ZSM-5
- on envoie dans l'unité d'isomérisation en phase gaz (ISOM-2) une seconde partie du flux (12), notée flux (15), pour obtenir un isomérat (16) qui est envoyé en mélange avec la charge fraiche (1) dans la colonne à distiller (S-1), ladite unité d'isomérisation en phase gaz (ISOM-2) fonctionnant aux conditions suivantes :
- température supérieure à 300°C, de préférence de 350°C à 480°C,
- pression inférieure à 4,0 MPa, et de préférence de 0,5 à 2,0 MPa,
- vitesse spatiale inférieure à 10 h⁻¹, de préférence comprise entre 0,5 h⁻¹ et 6 h⁻¹,
- rapport molaire hydrogène sur hydrocarbure inférieur à 10, et de préférence compris entre 3 et 6,
et le catalyseur utilisé dans ladite unité d'isomérisation ISOM-2 comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids, bornes incluses.

2. Procédé de production de paraxylène à haute pureté selon la revendication 1, dans lequel le catalyseur utilisé dans l'unité d'isomérisation (ISOM-2) renferme de 1% à 70% poids d'une zéolithe de type structural EUO (EU-1 par exemple) comprenant du silicium et au moins un élément T choisi de préférence parmi l'aluminium et le bore dont le rapport Si/T est compris entre 5 et 100.

3. Procédé de production de paraxylène à haute pureté selon la revendication 1, dans lequel la zéolithe faisant partie du catalyseur de l'unité d'isomérisation (ISOM-2) est sous forme hydrogène au moins en partie, et la teneur en sodium est telle que le ratio atomique Na/T est inférieur à 0,1.

4. Procédé de production de paraxylène à haute pureté selon la revendication 1, dans lequel le catalyseur de l'unité d'isomérisation (ISOM-2) peut contenir entre 0,01 et 2% poids d'étain ou d'indium, et du soufre à raison de 0,5 à 2 atomes par atome de métal du groupe VIII.

5. Procédé de production de paraxylène à haute pureté selon la revendication 1, dans lequel le nombre total de lits de l'unité de séparation (SMB) est compris entre 6 et 24 lits, et de manière préférée, entre 8 et 15 lits répartis sur un ou plusieurs adsorbeurs, le nombre de lits étant ajusté de manière à ce que chaque lit ait de une hauteur comprise entre 0,70 m et 1,40 m.

6. Procédé de production de paraxylène à haute pureté selon la revendication 1, dans lequel la répartition de la quantité de solide adsorbant dans chaque zone de l'unité de séparation (SMB) est la suivante :
• la quantité de solide adsorbant en zone 1 est de 17%±5%,
• la quantité de solide adsorbant en zone 2 est de 42%±5%,
• la quantité de solide adsorbant en zone 3 est de 25%±5%,
• la quantité de solide adsorbant en zone 4 est de 17%±5%,

7. Procédé de production de paraxylène à haute pureté selon la revendication 1, dans lequel on injecter le désorbant et la charge dans l'unité de séparation (SMB) dans un rapport volumétrique désorbant sur charge d'au plus 1,7/1, et de manière préférée compris entre 1,5/1 et 0,4/1, bornes incluses.

8. Procédé de production de paraxylène à haute pureté selon la revendication 1, dans lequel on extrait de l'unité de séparation (SMB) non plus un seul raffinat 9, mais deux raffinats (R1) et (R2), (R1) étant envoyé à l'unité d'isomérisation (ISOM-1) er (R2) étant envoyé dans l'unité d'isomérisation (ISOM-2).

## Patentansprüche

1. Verfahren zur Herstellung von Paraxylol mit hoher Reinheit aus einer Fraktion von Xylolen, enthaltend Ethylbenzol und C9+ Verbindungen, wobei das Verfahren eine Trenneinheit mit simuliertem mobilen Wanderbett (SMB) und zwei Isomerisierungseinheiten verwendet, wobei die eine (ISOM-1) in der Flüssigphase arbeitet, und die andere (ISOM-2) in der Gasphase arbeitet, wobei das Verfahren aus der Abfolge der folgenden Schritte besteht:
- die frische Charge (1) in Mischung mit dem Isomerisat (16), das aus der Isomerisierungseinheit (ISOM-2) in der Gasphase stammt, wird in die Destillationssäule (S-1) geschickt, aus der am Kopf ein Strom (3), der mit dem zweiten Isomerisat (14) gemischt wird, das aus der Isomerisierungseinheit (ISOM-1) in der Flüssigphase stammt, und am Boden ein Strom (4), der im Wesentlichen aus aromatischen C9 und C10 Verbindungen und gegebenenfalls aus Orthoxylol besteht, abgezogen werden,
- eine Trennung im simulierten mobilen Wanderbett des Stroms (5), der aus der Mischung der Ströme (3) und (14) erhalten wird, wird in einer Trenneinheit (SMB) durchgeführt, umfassend mindestens einen Adsorber, der eine Vielzahl von miteinander verbundenen Betten enthält und in geschlossener Schleife arbeitet, wobei die Trenneinheit mindestens vier Zonen umfasst, die auf folgende Weise definiert sind:
- die Zone 1 liegt zwischen dem Einbringen des Desorptionsmittels (11) und der Entnahme des Extrakts (6),
- die Zone 2 liegt zwischen der Entnahme des Extrakts (6) und dem Einbringen der Charge (5),
- die Zone 3 liegt zwischen dem Einbringen der Charge (5) und der Entnahme des Raffinats (9),
- die Zone 4 liegt zwischen der Entnahme des Raffinats (9) und dem Einbringen des Desorptionsmittels (11),
- der Extrakt (6) wird in eine Destillationssäule (EXT) geschickt, in der am Kopf eine Mischung von Paraxylol und Toluol durch die Leitung (7) abgezogen wird, und am Boden das Desorptionsmittel (8), das zur Trenneinheit (SMB) durch die Leitung (11) zurückgeschickt wird,
- das Raffinat (9) wird in eine Destillationssäule (RAF) geschickt, in der am Boden das Desorptionsmittel (10) abgezogen wird, das in die Trenneinheit (SMB) durch die Leitung (11) zurückgeschickt wird, und am Kopf eine Mischung von Metaxylol, Orthoxylol und Ethylbenzol, die durch eine Leitung (12) zu den Isomerisierungseinheiten (ISOM-1 und ISOM-2) geschickt wird,
- in eine Isomerisierungseinmheit in der Flüssigphase (ISOM-1) wird ein erster Teil des Stroms (12), der als Strom (13) bezeichnet wird, geschickt, um ein erstes Isomerisat (14) zu erhalten, das teilweise die Trenneinheit mit simuliertem mobilen Festbett (SMB) versorgt, wobei die Isomerisierungseinheit (ISOM-1) in der Flüssigphase unter den folgenden Bedingungen arbeitet:
- Temperatur kleiner als 300°C, vorzugsweise 200°C bis 260°C
- Druck kleiner als 4 MPa, vorzugsweise 2 bis 3 MPa
- Raumgeschwindigkeit pro Stunde (VVH) kleiner als 10 h⁻¹ (10 Liter pro Liter und pro Stunde), vorzugsweise zwischen 2 und 4 h⁻¹
- Katalysator, umfassend mindestens einen Zeolith mit Kanälen, deren Öffnung durch einen Ring mit 10 oder 12 Sauerstoffatomen (10 MR oder 12 MR) definiert ist, vorzugsweise ein Katalysator, umfassend mindestens einen Zeolith mit Kanälen, deren Öffnung durch einen Ring mit 10 Sauerstoffatomen (10 MR) definiert ist, und noch bevorzugter ein Katalysator, umfassend einen Zeolith des Typs ZSM-5
- in die Isomerisierungseinheit in der Gasphase (ISOM-2) wird ein zweiter Teil des Stroms (12), der als Strom (15) bezeichnet wird, geschickt, um ein Isomerisat (16) zu erhalten, das in Mischung mit der frischen Charge (1) in die Destillationssäule (S-1) geschickt wird, wobei die Isomerisierungseinheit in der Gasphase (ISOM-2) unter den folgenden Bedingungen arbeitet:
- Temperatur größer als 300°C, vorzugsweise von 350°C bis 480°C
- Druck kleiner als 4,0 MPa, und vorzugsweise von 0,5 bis 2,0 MPa
- Raumgeschwindigkeit kleiner als 10 h⁻¹, vorzugsweise zwischen 0,5 h⁻¹ und 6 h⁻¹
- Molverhältnis Wasserstoff zu Kohlenwasserstoff kleiner als 10, und vorzugsweise zwischen 3 und 6
und der Katalysator, der in der Isomerisierungseinheit ISOM-2 verwendet wird, umfasst mindestens einen Zeolith mit Kanälen, deren Öffnung durch einen Ring mit 10 oder 12 Sauerstoffatomen (10 MR oder 12 MR) definiert ist, und mindestens ein Metall der Gruppe VIII mit einem Gehalt zwischen 0,1 und 0,3 Gew.-%, Grenzen eingeschlossen.

2. Verfahren zur Herstellung von Paraxylol mit hoher Reinheit nach Anspruch 1, wobei der Katalysator, der in der Isomerisierungseinheit (ISOM-2) verwendet wird, 1 Gew.-% bis 70 Gew.-% eines Zeoliths des strukturellen Typs EUO (beispielsweise EU-1) umfasst, umfassend Silicium und mindestens ein Element T, vorzugsweise ausgewählt aus Aluminium und Bor, wobei das Verhältnis Si/T zwischen 5 und 100 liegt.

3. Verfahren zur Herstellung von Paraxylol mit hoher Reinheit nach Anspruch 1, wobei der Zeolith, der einen Teil des Katalysators der Isomerisierungseinheit (ISOM-2) bildet, mindestens teilweise in hydrierter Form ist, und der Gehalt an Natrium derart ist, dass das Atomverhältnis Na/T kleiner ist als 0,1.

4. Verfahren zur Herstellung von Paraxylol mit hoher Reinheit nach Anspruch 1, wobei der Katalysator der Isomerisierungseinheit (ISOM-2) zwischen 0,01 und 2 Gew.-% Zinn oder Indium, und Schwefel mit 0,5 bis 2 Atomen pro Atom eines Metalls der Gruppe VIII enthalten kann.

5. Verfahren zur Herstellung von Paraxylol mit hoher Reinheit nach Anspruch 1, wobei die Gesamtanzahl der Betten der Trenneinheit (SMB) zwischen 6 und 24 Betten, und bevorzugter zwischen 8 und 15 Betten liegt, die auf einem oder mehreren Adsorbern verteilt sind, wobei die Anzahl der Betten derart eingestellt wird, dass jedes Bett eine Höhe zwischen 0,70 m und 1,40 m aufweist.

6. Verfahren zur Herstellung von Paraxylol mit hoher Reinheit nach Anspruch 1, wobei die Verteilung der Menge an adsorbierendem Feststoff in jeder Zone der Trenneinheit (SMB) die folgende ist:
- die Menge an adsorbierendem Feststoff in der Zone 1 beträgt 17 % ± 5 %;
- die Menge an adsorbierendem Feststoff in der Zone 2 beträgt 42 % ± 5 %;
- die Menge an adsorbierendem Feststoff in der Zone 3 beträgt 25 % ± 5 %;
- die Menge an adsorbierendem Feststoff in der Zone 4 beträgt 17 % ± 5 %.

7. Verfahren zur Herstellung von Paraxylol mit hoher Reinheit nach Anspruch 1, wobei das Desorptionsmittel und die Charge in die Trenneinheit (SMB) in einem Volumenverhältnis von Desorptionsmittel zur Charge von höchstens 1,7/1, und bevorzugt zwischen 1,5/1 und 0,4/1, Grenzen eingeschlossen, eingebracht werden.

8. Verfahren zur Herstellung von Paraxylol mit hoher Reinheit nach Anspruch 1, wobei aus der Trenneinheit (SMB) nicht nur ein Raffinat 9, sondern zwei Raffinate (R1) und (R2) extrahiert werden, wobei (R1) zur Isomerisierungseinheit (ISOM-1) geschickt wird, und (R2) zur Isomerisierungseinheit (ISOM-2) geschickt wird.

## Claims

1. Process for the production of high-purity paraxylene based on a xylenes cut containing ethylbenzene and C9+ compounds, a process using one simulated moving bed separation unit (SMB) and two isomerization units, one (ISOM-1) operating in liquid phase, and the other (ISOM-2) operating in gas phase, the process consisting of the sequence of the following stages:
- the fresh feedstock (1) in a mixture with the isomerate (16) originating from the gas-phase isomerization unit (ISOM-2) is sent into the distillation column (S-1) from which a flow (3) which is mixed with the second isomerate (14) originating from the liquid-phase isomerization unit (ISOM-1) is removed at the top, and a flow (4) essentially constituted by C9 and C10 aromatic compounds and optionally orthoxylene is removed at the bottom.
- a simulated mobile bed separation of the flow (5) resulting from the mixture of the flows (3) and (14) is carried out in a separation unit (SMB) comprising at least one adsorber containing a plurality of interconnected beds and operating in a closed loop, said separation unit comprising at least four zones defined as follows:
- zone 1 comprised between the injection of the desorbent (11) and the draw-off of the extract (6),
- zone 2 comprised between the draw-off of the extract (6) and the injection of the feedstock (5),
- zone 3 comprised between the injection of the feedstock (5) and the draw-off of the intermediate raffinate (9),
- zone 4 comprised between the draw-off of the raffinate (9) and the injection of the desorbent (11),
- the extract 6 is sent into a distillation column (EXT), from which a mixture of paraxylene and toluene is drawn off at the top through the line (7) and the desorbent (8) which is sent back into the separation unit (SMB) is drawn off at the bottom through the line (11),
- the raffinate 9 is sent into a distillation column (RAF), from which the desorbent (10) which is sent back into the separation unit (SMB) through the line (11) is drawn off at the bottom, and a mixture of metaxylene, orthoxylene and ethylbenzene which is sent through a line (12) to the isomerization units (ISOM-1 et ISOM-2) is drawn off at the top,
- a first part of the flow (12), denoted flow (13), is sent into the liquid-phase isomerization unit (ISOM-1), in order to obtain a first isomerate (14), partially supplying the simulated moving bed separation unit (SMB), said isomerization unit (ISOM-1) operating in liquid phase under the following conditions:
- temperature less than 300°C, preferably 200°C to 260°C,
- pressure less than 4MPa, preferably 2 to 3 MPa,
- hourly space velocity (HSV) less than 10 h⁻¹ (10 litres per litre per hour), preferably comprised between 2 and 4 h⁻¹,
- catalyst comprising at least one zeolite having channels the opening of which is defined by a ring with 10 or 12 oxygen atoms (10 MR or 12 MR), preferentially a catalyst comprising at least one zeolite having channels the opening of which is defined by a ring with 10 oxygen atoms (10 MR), and even more preferably, a catalyst comprising a zeolite of ZSM-5 type.
- a second part of the flow (12), denoted flow (15), is sent into the gas-phase isomerization unit (ISOM-2), in order to obtain an isomerate (16), which is sent in a mixture with the fresh feedstock (1) into the distillation column (S-1), said gas-phase isomerization unit (ISOM-2) operating under the following conditions:
- temperature greater than 300°C, preferably from 350°C to 480°C,
- pressure less than 4.0 MPa and preferably from 0.5 to 2.0 MPa,
- hourly space velocity less than 10 h⁻¹, preferably comprised between 0.5 h⁻¹ and 6 h⁻¹,
- hydrogen to hydrocarbon molar ratio less than 10, and preferably comprised between 3 and 6.
and the catalyst used in said isomerization unit ISOM-2 comprising at least one zeolite having channels the opening of which is defined by a ring with 10 to 12 oxygen atoms (10 MR or 12 MR), and at least one group VIII metal at a content comprised between 0.1 and 0.3% by weight, inclusive.

2. Process for the production of high-purity paraxylene according to claim 1, in which the catalyst used in the isomerization unit (ISOM-2) contains from 1 to 70% by weight of a zeolite of the EUO structure type (EU-1 for example) comprising silicon and at least one element T preferably selected from aluminium and boron, the Si/T ratio of which is comprised between 5 and 100.

3. Process for the production of high-purity paraxylene according to claim 1, in which the zeolite forming part of the isomerization unit (ISOM-2) is at least partially in the form of hydrogen, and the sodium content is such that the Na/T atomic ratio is less than 0.1.

4. Process for the production of high-purity paraxylene according to claim 1, in which the catalyst of the isomerization unit (ISOM-2) can contain between 0.01 and 2% by weight of tin or indium, and sulphur at a content of 0.5 to 2 atoms per atom of the group VIII metal.

5. Process for the production of high-purity paraxylene according to claim 1, in which the total number of beds of the separation unit (SMB) is comprised between 6 and 24 beds, and preferably between 8 and 15 beds, distributed over one or more adsorbers, the number of beds being adjusted so that each bed has a height comprised between 0.70 m and 1.40 m.

6. Process for the production of high-purity paraxylene according to claim 1, in which the distribution of the quantity of solid adsorbent in each zone of the separation unit (SMB) is as follows:
• the quantity of solid adsorbent in zone 1 is 17%±5%,
• the quantity of solid adsorbent in zone 2 is 42%±5%,
• the quantity of solid adsorbent in zone 3 is 25%±5%,
• the quantity of solid adsorbent in zone 4 is 17%±5%,

7. Process for the production of high-purity paraxylene according to claim 1, in which the desorbent and the feedstock are injected into the separation unit (SMB) in a desorbent to feedstock ratio by volume of at most 1.7/1 and preferably comprised between 1.5/1 and 0.4/1, inclusive.

8. Process for the production of high-purity paraxylene according to claim 1, in which not one but two raffinates (R1) and (R2) are extracted from the separation unit (SMB), (R1) being sent to the isomerization unit (ISOM-1) and (R2) being sent into the isomerization unit (ISOM-2).
